# EUROPÄISCHE PATENTANMELDUNG

(11) **EP 1 532 956 A2**
(43) Veröffentlichungstag der Anmeldung: **25.05.2005**
(21) Anmeldenummer: 04022815.7
(22) Anmeldetag: 24.09.2004
(51) Int. Cl.: A61G 13/02, G06F 19/00

(54) **Verfahren zur Vorbereitung von für die Durchführung von medizinischen oder chirurgischen Eingriffen bestimmten Geräten**

(30) Priorität: 18.11.2003 DE 10353846
(71) Anmelder: MAQUET GmbH & Co. KG, 76437 Rastatt (DE)
(72) Erfinder: Wyslucha, Ulrich, 76189 Karlsruhe (DE); Welsch, Michael, 76571 Gaggenau (DE)
(74) Vertreter: Schaumburg, Thoenes, Thurn, Landskron

(57) **Zusammenfassung**

Ein Verfahren zur Vorbereitung und/oder Bereitstellung von für die Durchführung von medizinischen oder chirurgischen Eingriffen bestimmten Geräten ist dadurch gekennzeichnet, daß an einem mit einer Datenverarbeitungseinrichtung verbundenen, mindestens eine Anzeigeeinrichtung und ein Bedienungsfeld umfassenden Arbeitsplatz ein durchzuführender Eingriff über das Bedienungsfeld aufgerufen wird, worauf an der Anzeigeeinrichtung das hierfür geeignete Gerät oder die geeigneten Geräte und/oder ihre Einstellung bzw. ihre Positionierung relativ zueinander angezeigt werden.

## Beschreibung

Die Erfindung betrifft ein Verfahren zur Vorbereitung und/oder Bereitstellung von für die Durchführung von medizinischen oder chirurgischen Eingriffen bestimmten Geräten.

Die Darstellung von Patientenlagerungen für einen bestimmten medizinischen oder chirurgischen Eingriff erfolgt üblicherweise als ein Bild in der Endposition mit erklärendem Text. Zwischenschritte werden in der Regel nicht dargestellt. Dazu gibt es in Ordnern gesammelte Beschreibungen, die mehr oder weniger systematisch aufgebaut sind und in denen auch das für den jeweiligen Eingriff benötigte Zubehör und dessen Handhabung beschrieben ist. Die Nutzung dieser Informationen zur Vorbereitung eines bestimmten Eingriffs setzt große Erfahrung und Vertrautheit mit dem jeweiligen Gerät voraus, um den Eingriff optimal vorbereiten zu können.

Der Erfindung liegt die Aufgabe zugrunde, ein Verfahren der eingangs genannten Art anzugeben, das die Vorbereitung und Bereitstellung der für einen Eingriff gewünschten oder erforderlichen Geräte erleichtert und gleichzeitig die Sicherheit für eine optimale Vorbereitung erhöht.

Diese Aufgabe wird erfindungsgemäß dadurch gelöst, daß an einem mit einem Computer verbundenen, mindestens eine Anzeigeeinrichtung und ein Bedienungsfeld umfassenden Arbeitsplatz ein durchzuführender Eingriff über das Bedienungsfeld aufgerufen wird, worauf an der Anzeigeeinrichtung das hierfür geeignete oder erforderliche Gerät oder die entsprechenden Geräte und/oder ihre Einstellung bzw. ihre Positionierung relativ zueinander angezeigt werden. Ist beispielsweise eines der Geräte ein Operationstisch, dessen Patientenlagerfläche eine Mehrzahl von im Raum verstellbaren Lagerflächenabschnitten hat, so kann die für einen Eingriff erforderliche Stellung der jeweiligen Lagerflächenabschnitte angezeigt werden. Ferner können die für den Eingriff erforderlichen Zusatzgeräte und -instrumente angezeigt werden, wobei dies in Bild und/oder Wort erfolgen kann.

Gleichzeitig kann die Möglichkeit bestehen, die Position der Zusatzgeräte an dem Operationstisch anzuzeigen.

Besonders hilfreich ist es, wenn das Erreichen einer bestimmten Einstellung und/oder Position eines Gerätes oder Geräteteiles schrittweise dargestellt wird, indem beispielsweise die Schritte in einer bestimmten zeitlichen Folge wiedergegeben werden oder einzeln von einer Bedienungsperson durch Eingaben an den Bedienungsfeld fortgeschaltet oder abgerufen werden. Auf diese Weise kann sich die Bedienungsperson die einzelnen Schritte und den Ablauf der für das Erreichen einer bestimmten Endeinstellung erforderlichen Maßnahmen einprägen. Hierzu ist es hilfreich, wenn die Einstellungen oder Positionen nicht nur bildlich dargestellt werden, sondern die Gesamtheit der Schritte bis zum Erreichen einer Endposition oder -einstellung in einzelnen Bildern gleichzeitig auf der Anzeigeeinrichtung dargestellt wird.

Wenn sich das Erreichen einer bestimmten Einstellung oder Endposition nicht aufgrund der Ausgestaltung des Gerätes von selbst versteht, kann es zweckmäßig sein, die zum Erreichen einer bestimmten Einstellung oder Position erforderlichen Maßnahmen ebenfalls anzuzeigen. Bei komplizierteren Einstellungen kann es besonders hilfreich sein, wenn die Geräte und/oder ihre Einstellungen und/oder Positionierungen in zeitlich aufeinanderfolgenden Bildern dargestellt werden, wobei man eine lockere in größeren Abständen ablaufende Bildfolge oder einen Film wählen kann.

Bei einer bevorzugten Ausführungsform der Erfindung ist der Arbeitsplatz mit einem einzustellenden Gerät, beispielsweise einem Operationstisch verbunden, wobei dieser entsprechend den angezeigten Einstellungen und Positionen automatisch angesteuert wird. Ferner kann die erfindungsgemäße Lösung auch dafür genutzt werden, die für einen Eingriff tatsächlich verwendeten Geräte und/oder Einstellungen bzw. Positionierungen zu erfassen und abzuspeichern. Die gespeicherten Daten können einem Patienten zugeordnet werden, so daß auf diese Weise eine Dokumentation der für den Eingriff verwendeten Geräte und Instrumente sowie ihrer Handhabung möglich wird. Das Eingeben der Daten kann entweder durch eine direkte Verbindung zwischen dem Arbeitsplatz und dem betreffenden Gerät erfolgen, indem beispielsweise die Einstellungen des Gerätes über Sensoren erfaßt werden. Das Vorhandensein der erforderlichen oder gewünschten Zusatzgeräte sowie ihrer Position und Einstellung kann über Transponder erfolgen. Ebenso besteht aber auch die Möglichkeit, die verwendeten Geräte und ihre Einstellungen bzw. Positionen mittels einer Stehbildoder Videokamera zu erfassen und die erforderlichen Daten dem Computer zuzuleiten, sei es über eine direkte Verbindung oder über ein transportables Speichermedium.

Die Erfindung betrifft ferner eine Computersoftware, umfassend Befehle und Daten in codierter Form, die nach dem Laden der Computersoftware ein Computersystem veranlassen, die oben beschriebenen Verfahrensschritte auszuführen. Die Computersoftware umfaßt in der Regel eine Bedieneroberfläche, d.h. ein user interface, und eine Datenbank. Die Datenbank kann einen Speicher für erste Daten und Instruktionen umfassen, die durch einen ersten Zugriffsschlüssel geschützt sind, sowie mindestens einen zweiten Speicher, der zur Aufnahme zweiter Daten und Instruktionen bestimmt ist. Auf diese Weise besteht die Möglichkeit, nicht variable Daten, die beispielsweise von dem Hersteller eines Operationstischsystems vorgegeben werden, in dem ersten Speicher abzulegen, während der zweite Speicher dem Anwender die Möglichkeit gibt, eigene Einstellungsvorschläge und das für einen Eingriff erforderliche Zubehör zu spezifizieren und somit die Computersoftware seinen eigenen Vorstellungen anzupassen.

Die erfindungsgemäße Computersoftware bietet somit die Möglichkeit, auf der Anzeigevorrichtung, d.h. in der Regel einem Bildschirm, die einzelnen Schritte zum Einrichten eines Operationstisches und der dabei benötigten Zubehörteile darzustellen. Der Lagerungsfortschritt wird damit sichtbar und nachvollziehbar. Für die Darstellung können Bilder, Videos und Texte verwendet werden. Dabei kann es zweckmäßig sein, die Texte auch in beliebig vielen Sprachen zu verwalten. Für jede Patientenlagerung kann eine Stückliste des benötigten Zubehörs ausgegeben werden. Die einzelnen Lagerungsschritte können darüber hinaus individuell durch Notizen und Bemerkungen des Operationspersonals ergänzt werden. Dadurch kann das Benutzerwissen dokumentiert und verfügbar gemacht werden. Ggf. kann der Benutzer auch bestimmte Favoriten definieren, wenn der Zugriff auf häufig benötigte Elemente beschleunigt werden soll.

Der wesentliche Vorteil der erfindungsgemäßen Lösung besteht darin, daß komplexe Zusammenhänge aufgelöst dargestellt werden und daß auf Information schnell und sicher zugegriffen werden kann. Gleichzeitig bietet sich auch die Möglichkeit, das Programm mit einem Programm für die Lagerhaltung des für eine Operation benötigten Zubehörs zu verbinden, so daß das Personal beispielsweise rechtzeitig eine Auskunft über das verfügbare Material und seinen Zustand, beispielsweise den Sterilisierungszustand erhält.

Die erfindungsgemäße Software kann ohne weiteres an mehreren Arbeitsplätzen eingesetzt werden, die beispielsweise als Klienten mit einem Datenbankserver verbunden sind.

Die Erfindung betrifft somit ferner ein Computersystem mit mindestens einem Computer, einer Anzeigevorrichtung und einem Bedienfeld sowie einer Computersoftware der oben beschriebenen Art. Das Bedienfeld kann beispielsweise ein Touchscreen sein, der sich insbesondere für die Verwendung in einem Operationssaal eignet. Das System kann direkt mit einem einzustellenden Gerät, beispielsweise einem Operationstisch verbunden sein, so daß bei Aufruf eines bestimmten Eingriffes die erforderlichen Einstellungen und das benötigte Zubehör nicht nur angezeigt werden, sondern daß über den Computer auch der Operationstisch direkt angesteuert werden kann, um die im Programm vorgesehenen Einstellungen vorzunehmen. Umgekehrt kann die jeweils vorgenommene Einstellung zu Dokumentationszwecken abgespeichert werden.

Weitere Merkmale und Vorteile der Erfindung ergeben sich aus den Unteransprüchen und der folgenden Beschreibung, welche in Verbindung mit den beigefügten Figuren die Erfindung anhand von Ausführungsbeispielen erläutert. Es zeigen:
- Fig.1: eine schematische Darstellung eines zur Durchführung des erfindungsgemäßen Verfahrens geeigneten Computersystems in Verbindung mit einem schematisch dargestellten Operationstisch und
- Fig.2 bis 9: verschiedene Abbildungen auf einem Bildschirm zur Erläuterung von einigen Funktionen des erfindungsgemäßen Computerprogramms.

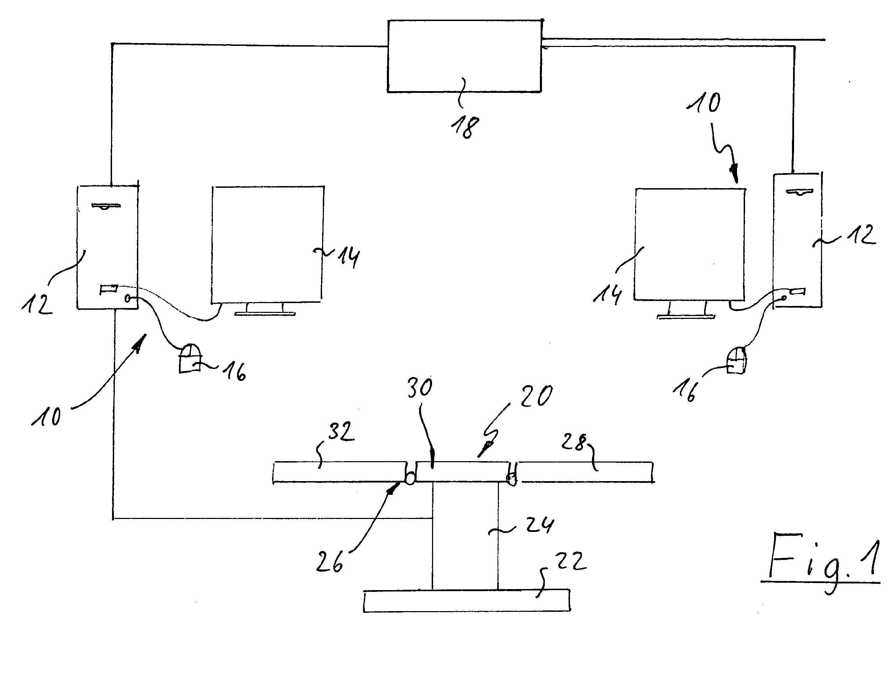

In Fig.1 ist mit 10 jeweils ein Arbeitsplatz bezeichnet, der einen PC 12 und einen mit diesem verbundenen Bildschirm 14 umfaßt. Der Bildschirm 14 kann als Touchscreen ausgebildet sein. Alternativ kann der PC 12 mit einer Maus 16 oder einer nicht dargestellten Tastatur verbunden sein. Der PC 12 ist mit einem Datenbankserver 18 verbunden. Ferner ist in dem dargestellten Ausführungsbeispiel der PC 12 direkt mit einem Operationstisch 20 verbunden, um diesen zu steuern. Der Operationstisch 20 hat einen Fuß 22 mit einer Tragsäule 24 und einer Patientenlagerfläche 26, die eine Mehrzahl von Abschnitten 28, 30, 32 umfaßt, die relativ zueinander und zur Tragsäule 24 mittels geeigneter Motoren verstellbar sind. Solche Operationstische sind bekannt und brauchen im Detail nicht mehr erläutert zu werden.

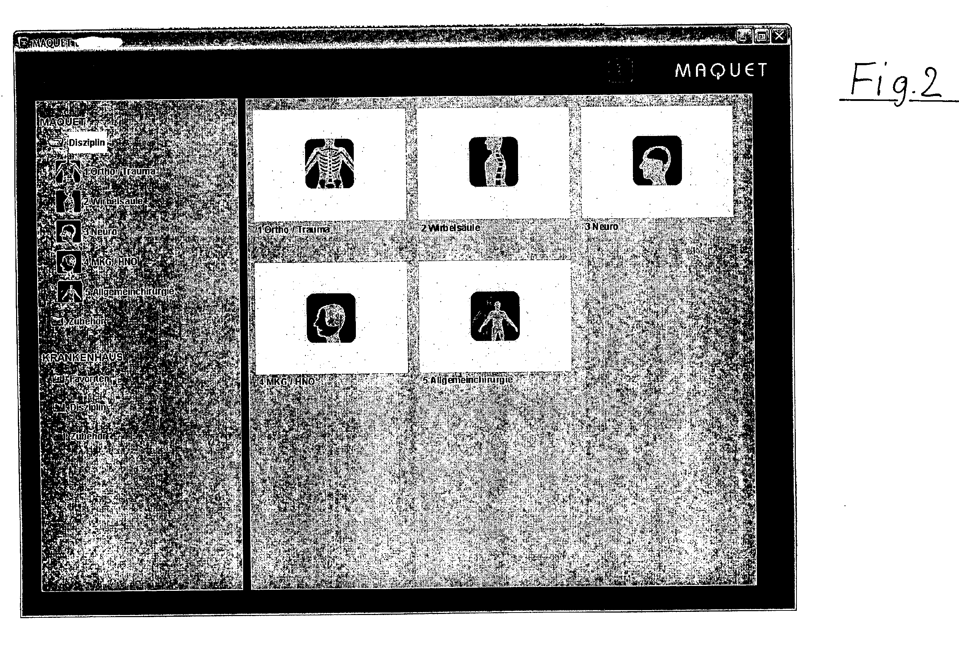

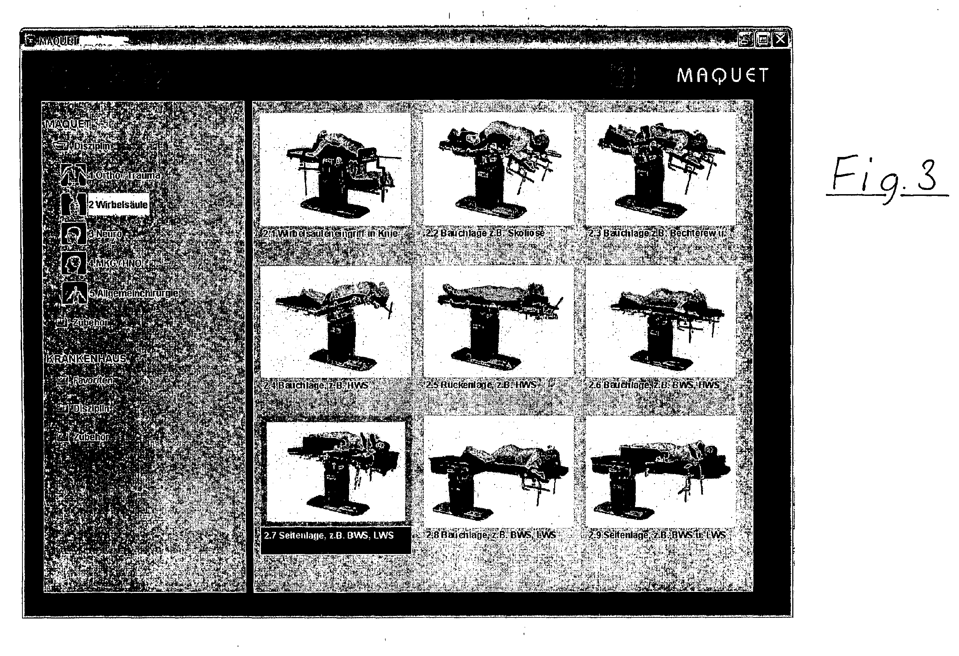

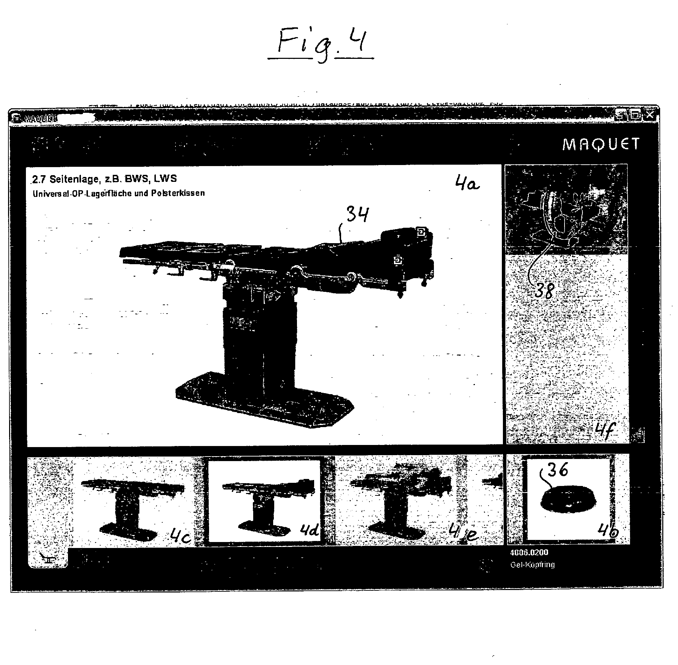

Das erfindungsgemäße Computerprogramm ermöglicht es, die für einen bestimmten Eingriff bestimmten Geräte und ihre Einstellung dem Bedienungspersonal darzustellen und zu erläutern. Beispiele hierfür sind in den Fig.2 bis 8 wiedergegeben. So zeigt Fig.2 ein erstes Bild auf dem Bildschirm mit einer Auswahl der möglichen Disziplinen. Wird eine Disziplin, beispielsweise die Wirbelsäulenchirurgie ausgewählt, so werden gemäß Fig.3 verschiedene Lagerungsmöglichkeiten für unterschiedliche Eingriffe dargestellt, so daß der Anwender sich die geeignete Lagerungsmöglichkeit auswählen kann. In Fig.3 ist dies die Seitenlage nach Abb.2.7. Bei Auswahl dieser Seitenlage wird ihm nun in Fig.4 der Operationstisch in dem großen Bild 4A mit einem Keilkissen 34 für die Seitenlage und einem Gelkopfring 36 dargestellt, der als Zubehörteil noch einmal in dem kleinen 4B dargestellt ist. Die kleinen Bilder 4C bis 4E unterhalb des großen Bildes 4A zeigen den Operationstisch zunächst ohne Zubehör (4C), mit dem Zubehör wie in 4A (4D) und mit dem Patienten in Seitenlage (4E). Bild 4F schließlich zeigt den Patienten in Verbindung mit einem C-Bogen 38.

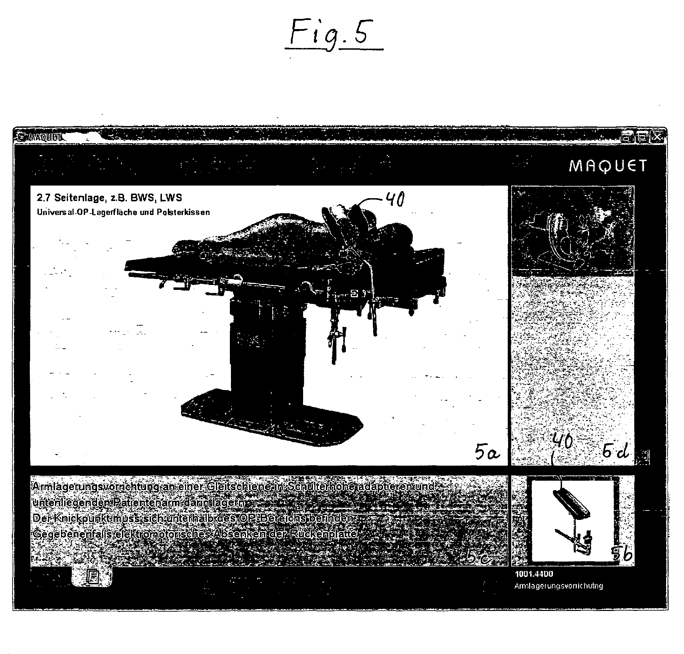

Fig.5 zeigt im Abschnitt 5A den Operationstisch gemäß Fig.4 nun mit dem Patienten und einer Armlagerungsvorrichtung 40, die in dem Bildabschnitt 5B einzeln dargestellt ist. Der Bildabschnitt 5C gibt Erläuterungen für die Montage der Armlagerungsvorrichtung. Der Bildabschnitt 5D entspricht dem Bildabschnitt 4F.

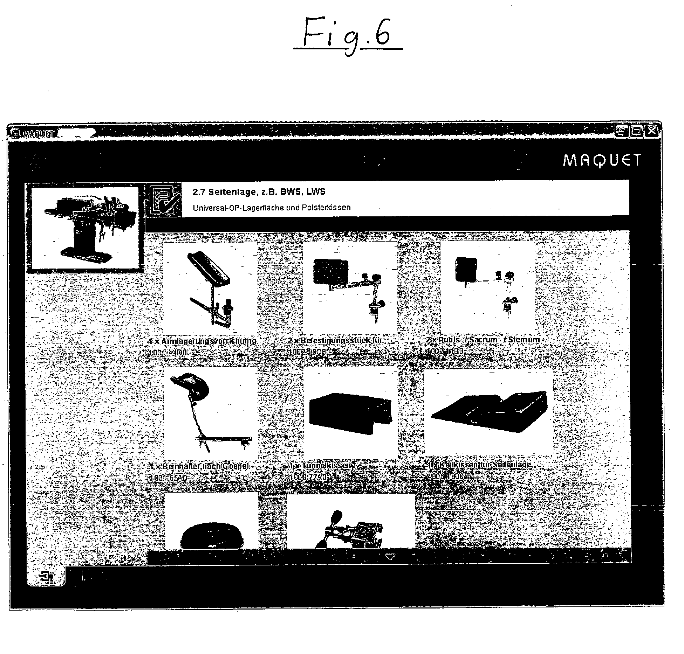

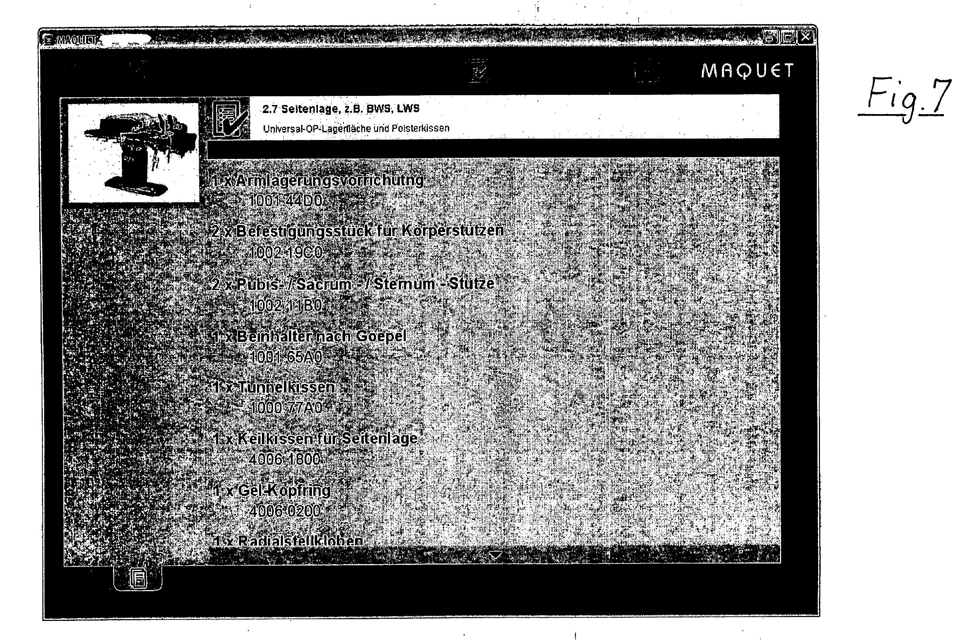

Fig.6 zeigt das für die Seitenlage erforderliche Gesamtzubehör mit Artikelnummern und Anzahl. Fig.7 zeigt die gleichen Zubehörteile in Form einer Liste.

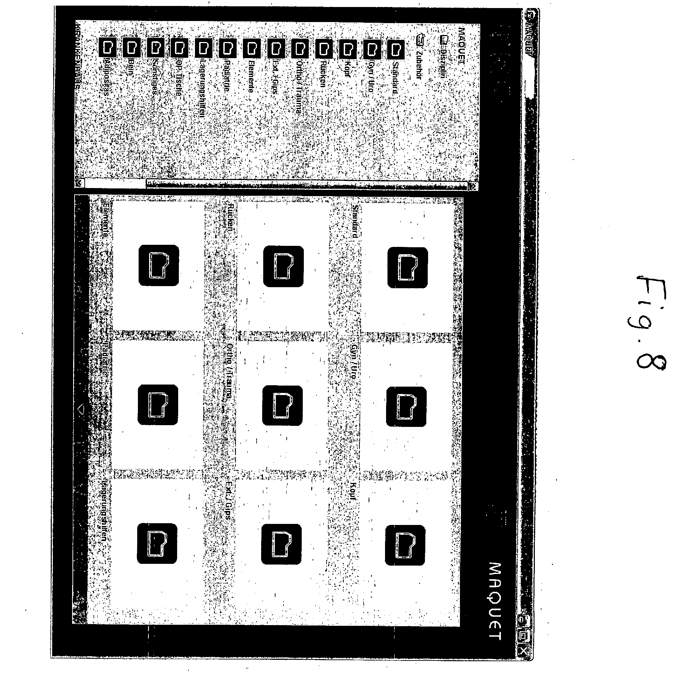

Fig.8 zeigt eine Abbildung, in der der Anwender verschiedene Zubehörteile auswählen kann.

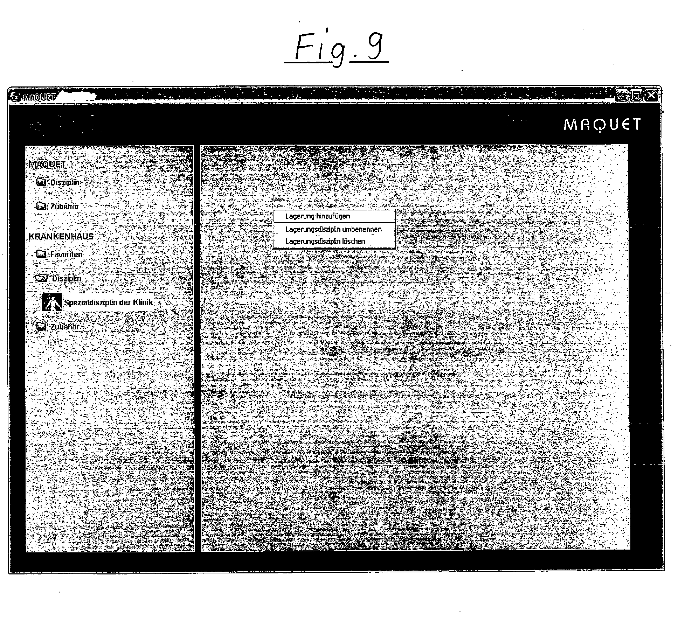

Fig.9 schließlich zeigt ein Bild, demzufolge der Anwender das Programm durch eigene Daten ergänzen oder ändern kann, um es seinen speziellen Bedürfnissen anzupassen oder durch eigene Erfahrungen und Vorschläge zu ergänzen.

Ist ein Eingriff durch die Instruktionen des Computerprogramms entsprechend vorbereitet, so kann der Operationstisch in Übereinstimmung mit diesem Programm direkt angesteuert und eingestellt werden, wobei beispielsweise Signale gegeben werden, wann ein bestimmtes Zubehörteil angebaut und eingestellt werden soll, worauf nach einer entsprechenden Quittung durch den Anwender das Programm fortschreitet und die nächste Einstellung bekannt gibt. Es kann auch über diese fortschreitende Einstellung ein Lagerungsprotokoll aufgenommen werden. Ggf. können Zubehörteile auch automatisch registriert werden über entsprechende Transponder oder Sensoren. Erstellte Lagerungsprotokolle können ausgedruckt werden oder an andere Programme überspielt werden, um beispielsweise das entsprechende Protokoll in einem Patientenverwaltungssystem dem jeweiligen Patienten zuordnen zu können.

Das erfindungsgemäße Programm und System hat eine Reihe von Vorteilen. So bietet es die Möglichkeit zur interaktiven Schulung und zur Weiterbildung des Klinikpersonals. Der Lagerungsaufbau für einen Patienten kann Schritt für Schritt ausgeführt werden, wobei die Prozesse optimiert werden und sichergestellt wird, daß kein Schritt ausgelassen wird. Dadurch ergibt sich auch eine höhere Qualitätssicherung. Gleichzeitig können die Vorgänge im Operationssaal besser dokumentiert werden. Es können die vom Hersteller für ein bestimmtes System vorgesehenen Lagerungsvorschläge optimal umgesetzt werden. Gleichzeitig kann aber die jeweilige Klinik eigene Lagerungen und Zubehöre in dem Programm speichern, um so für einen bestimmten Standard innerhalb der eigenen Klinik zu sorgen.

Das erfindungsgemäße Programm ist auf herkömmlichen Datenverarbeitungsanlagen lauffähig, beispielsweise einem PC, und kann für mehrere Anwender gleichzeitig genutzt werden. Es kann über eine Tastatur oder Maus oder einen Touchscreen bedient werden.

Das erfindungsgemäße Programm ermöglicht eine Vernetzung von Informationen, z.B. eine Auflistung aller Patientenlagerungen, die ein bestimmtes Zubehör verwenden. Es können bestimmte Gebrauchsanleitungen des Herstellers eines Systems eingespeist werden, die auch auf einfache Weise dem jeweiligen technischen Stand entsprechend erweiterbar sind, so daß sichergestellt ist, daß die jeweils aktuelle Version der Gebrauchsanleitung vorliegt.

Mit dem erfindungsgemäßen System ist es auf einfache Weise möglich, sicherzustellen, daß das Personal die für eine bestimmte Patientenlagerung erforderlichen Teile vollständig ermitteln und bereitstellen kann. Gleichzeitig werden die Teile dargestellt und mit den korrekten Namen benannt, was die Kommunikation innerhalb des Personals erleichtert.

## Patentansprüche

1. Verfahren zur Vorbereitung und/oder Bereitstellung von für die Durchführung von medizinischen oder chirurgischen Eingriffen bestimmten Geräten, **dadurch gekennzeichnet, daß** an einem mit einer Datenverarbeitungseinrichtung verbundenen, mindestens eine Anzeigeeinrichtung und ein Bedienungsfeld umfassenden Arbeitsplatz ein durchzuführender Eingriff über das Bedienungsfeld aufgerufen wird, worauf an der Anzeigeeinrichtung das hierfür geeignete Gerät oder die geeigneten Geräte und/oder ihre Einstellung bzw. ihre Positionierung relativ zueinander angezeigt werden.

2. Verfahren nach Anspruch 1, **dadurch gekennzeichnet, daß** eines der Geräte ein Operationstisch ist, dessen Patientenlagerfläche eine Mehrzahl von im Raum verstellbaren Lagerflächenabschnitten umfaßt, und daß die für einen Eingriff erforderlichen Stellungen der Lagerflächenabschnitte angezeigt werden.

3. Verfahren nach Anspruch 2, **dadurch gekennzeichnet, daß** die für den Eingriff erforderlichen Zusatzgeräte und -instrumente angezeigt werden.

4. Verfahren nach Anspruch 3, **dadurch gekennzeichnet, daß** die Position der Zusatzgeräte an dem Operationstisch angezeigt wird.

5. Verfahren nach einem der Ansprüche 1 bis 4, **dadurch gekennzeichnet, daß** das Erreichen einer bestimmten Einstellung oder Position eines Gerätes oder Geräteteiles schrittweise dargestellt wird.

6. Verfahren nach Anspruch 5, **dadurch gekennzeichnet, daß** die Schritte durch Eingabe am Bedienungsfeld fortgeschaltet werden.

7. Verfahren nach einem der Ansprüche 1 bis 6, **dadurch gekennzeichnet, daß** die Einstellungen oder Positionen bildlich dargestellt werden.

8. Verfahren nach einem der Ansprüche 5 bis 7, **dadurch gekennzeichnet, daß** die Gesamtheit der Schritte bis zum Erreichen einer Endposition oder -einstellung in einzelnen Bildern gleichzeitig dargestellt wird.

9. Verfahren nach einem der Ansprüche 1 bis 8, **dadurch gekennzeichnet, daß** die zum Erreichen einer bestimmten Einstellung oder Position erforderlichen Maßnahmen angezeigt werden.

10. Verfahren nach einem der Ansprüche 1 bis 7 und 9, **dadurch gekennzeichnet, daß** die Geräte und/oder ihre Einstellung und/oder Positionierung in zeitlich aufeinanderfolgenden Bildern dargestellt werden..

11. Verfahren nach einem der Ansprüche 1 bis 10, **dadurch gekennzeichnet, daß** bei Verbindung des Arbeitsplatzes mit einem einstellbaren Gerät, insbesondere einem Operationstisch, dieses entsprechend den angezeigten Einstellungen und Positionen automatisch angesteuert wird.

12. Verfahren nach einem der Ansprüche 1 bis 11, **dadurch gekennzeichnet, daß** die für einen Eingriff tatsächlich verwendeten Geräte und Einstellungen bzw. Positionierungen erfaßt und gespeichert werden.

13. Verfahren nach Anspruch 12, **dadurch gekennzeichnet, daß** die gespeicherten Daten einem Patienten zugeordnet werden.

14. Computersoftware, umfassend Befehle und Daten in codierter Form, die nach dem Laden der Computersoftware ein Computersystem veranlassen, die in einem der Ansprüche 1 bis 13 aufgeführten Schritte auszuführen.

15. Computersoftware nach Anspruch 14, **dadurch gekennzeichnet, daß** sie auf einem Speichermedium gespeichert ist.

16. Computersoftware nach Anspruch 14 oder 15, **dadurch gekennzeichnet, daß** sie eine Bedieneroberfläche und/oder eine Datenbank umfaßt.

17. Computersoftware nach Anspruch 16, **dadurch gekennzeichnet, daß** die Datenbank einen Speicher mit ersten Daten und Instruktionen umfaßt, die durch einen ersten Zugriffsschlüssel geschützt sind, und daß die Datenbank mindestens einen zweiten Speicher hat, der zur Aufnahme weiterer Daten und Instruktionen bestimmt ist.

18. Computersystem mit mindestens einem Computer, einer Anzeigevorrichtung und einem Bedienfeld sowie einer Computersoftware nach einem der Ansprüche 14 bis 17.

19. Computersystem nach Anspruch 18, **dadurch gekennzeichnet, daß** das Bedienfeld auf einem die Anzeigevorrichtung bildenden Touchscreen ausgebildet ist.

20. Computersystem nach Anspruch 18 oder 19, **dadurch gekennzeichnet, daß** es mit einem einzustellenden Gerät verbunden ist.

21. Computersystem nach einem der Ansprüche 18 bis 20, **dadurch gekennzeichnet, daß** die Einstellung bzw. die Position eines einzustellenden Gerätes oder mit ihm verbundener Zubehörteile durch Sensoren erfaßbar und in dem Computersystem speicherbar sind.

22. Computersystem nach Anspruch 20, **dadurch gekennzeichnet, daß** die Sensoren mit dem einzustellenden oder zu positionierenden Teilen verbundene Transponder sind.

23. Computersystem nach einem der Ansprüche 18 bis 22, **dadurch gekennzeichnet, daß** die Sensoren mindestens eine Video- und/oder Stehbildkamera umfassen.
